# FASCICULE DE BREVET EUROPEEN

(11) **EP 0 717 748 B1**
(45) Date de publication et mention de la délivrance du brevet: **17.12.1997**
(21) Numéro de dépôt: 94926973.2
(22) Date de dépôt: 09.09.1994
(51) Int. Cl.: C07H 19/04, A61K 31/70, C07D 405/04, C07D 473/00, A61K 31/505, A61K 31/52

(54) **COMPOSES 2' OU 3'-DEOXY- ET 2', 3'-DIDEOXY-BETA-L-PENTOFURANONUCLEOSIDES, PROCEDE DE PREPARATION ET APPLICATION THERAPEUTIQUE, NOTAMMENT ANTI-VIRALE**
2' ODER 3' -DEOXY- UND 2' -DIDEOXY-BETA-L-PENTAFURANONUKLEOSIDE, VERFAHREN ZUR HERSTELLUNG UND ANWENDUNG IN DER THERAPIE, INSBESONDERE ALS ANTIVIRALE WIRKSTOFFE
2' OR 3'-DEOXY AND 2'-DIDEOXY-BETA-L-PENTAFURANONUCLEOSIDE COMPOUNDS, METHOD OF PREPARATION AND APPLICATION IN THERARY, ESPECIALLY AS ANTI-VIRAL AGENTS

(30) Priorité: 10.09.1993 FR 9310798
(43) Date de publication de la demande: 26.06.1996
(73) Titulaire: CENTRE NATIONAL DE LA RECHERCHE SCIENTIFIQUE (CNRS), 75794 Paris Cédex 16 (FR)
(72) Inventeur: GOSSELIN, Gilles, Bâtiment F1, F-34080 Montpellier (FR); IMBACH, Jean-Louis, F-34000 Montpellier (FR); AUBERTIN, Anne-Marie, F-67000 Strasbourg (FR); SOMMADOSSI, Jean-Pierre, Birmingham, AL 35242 (US); SCHINAZI, Raymond, F., Decatur, GA 30033 (US)
(74) Mandataire: Warcoin, Jacques
(86) Numéro de dépôt international: FR9401066
(87) Numéro de publication internationale: WO9507287

(56) Documents cités:
- EP-A- 0 285 884
- EP-A- 0 352 248
- WO-A-92/06102
- WO-A-92/08727
- US-A- 3 116 282
- US-A- 3 553 192
- NUCLEIC ACIDS RESEARCH., vol.19, no.15, 1991, ARLINGTON, VIRGINIA US pages 4067 - 4074 U.ASSELINE ET AL. 'Synthesis and Physicochemical Properties of Oligonucleotides built with either alpha-L or beta-L Nucleotides Units and Covalently Linked to an Acridine Derivative.'
- JOURNAL OF MEDICINAL CHEMISTRY, vol.35, no.22, 1992, WASHINGTON US pages 4214 - 4220 S.SPADARI ET AL. 'L-Thymidine Is Phosphorylated by Herpes Simplex Virus Type 1 Thymidine Kinase and Inhibits Viral Growth.' cité dans la demande
- JOURNAL OF ORGANIC CHEMISTRY., vol.53, no.20, 1988, EASTON US pages 4780 - 4786 M.OKABE ET AL. 'Synthesis of the Dideoxynucleosides ddC and CNT from Glutamic Acid, Ribonolactone , and Pyrimidine Bases.' cité dans la demande

## Description

La présente invention a pour objet un procédé de préparation stéréospécifique de composés 2' ou 3' déoxy et 2', 3'-didéoxy-β-L-pentofuranonucléosides.

La présente invention concerne également des composés 2' ou 3' déoxy et 2', 3' didéoxy-β-L-pentofuranonucléosides.

La présente invention concerne enfin l'utilisation de ces composés à titre de médicaments et notamment à titre d'agents anti-viraux.

A ce jour, la synthèse et l'évaluation biologique des analogues nucléosidiques de configuration L ont fait l'objet de certains travaux, mais jusqu'à récemment, les activités de la plupart des nucléosides étaient uniquement associées à celles de leurs isomères D [A. Holy, in Synthesis, Structure and Chemistry of Transfer Ribonucléic Acids and their Components (Proceedings of the International Conference Held in Dymaczewo near Poznan, Poland on September 13-17, 1976), Polish Academy of Sciences, Poznan, 1976, p. 134, et références citées]. Cependant, récemment, la β-L-Thymidine [S. Spadari, G. Mage, F. Focher, G. Ciarrocchi, R. Manserwigi, F. Arcamone, M. Capobianco, A. Carcuro, F. Colonna, S. Iotti et A. Garbesi, J. Med. Chem. 35, 4214 (1992)] et la 2', 3'-didéoxy-β-L-cytidine (β-L-DDC) [M. M. Mansuri, V. Farina, J.E Starret Jr., D.A. Benigni, V. Brankovan et J.C. Martin, Bioorg. Med. Chem. Letters, 1, 65 (1991)] ont été montrées comme exerçant une activité antivirale relativement limitée en cultures cellulaires contre, respectivement, les virus herpès simplex (HSV) et le virus de l'immunodéficience humaine (HIV), ce dernier étant l'agent causal du syndrome d'immunodéficience acquise (SIDA).

En réalité, la β-L-DDC a été préalablement rapportée de façon contradictoire comme, d'une part, ne présentant aucune activité contre le HIV [M. Okabe, R.C. Sun, S. Y.-K. Tam, L.J. Todaro et D.L. Coffen, J. Org. Chem. 53, 4780 (1988)] et, d'autre part, exhibant une activité modérée toujours contre le HIV [IC₅₀ = O,66 10⁻⁶M en culture cellulaire CEM: M.M. Mansuri, Y. Farina, J.E. Starret Jr., D.A. Benigni, V. Brankovan et J.C. Martin, Bioorg. Med. Chem. Letters, 1, 65 (1991)].

D'autre part, des analogues d'isomères L de l'AZT ont été testés et sont apparus comme inactifs comme agent anti-HIV [J. Org. Chem. 56, 3591 (1991)].

C'est pourquoi en série isomérique β-L des analogues nucléotidiques de type dioxolanyl [H.O. Kim, R.F. Schinazi, K. Shanmuganathan, L.S. Jeong, J.W. Beach, S. Nampalli, D.L. Cannon et C.K. Chu, J. Med. Chem., 36, 519 (1993) et références citées] et de type oxathiolanyl [L.S. Jeong, R.F. Schinazi, J.W. Beach, H.O. KIM, S. Mampalli, K. Shanmuganathan, A.J. Alues, A. McMillan, C.K. Chu et R. Mathis, J. Med. Chem. 36, 181 (1993), et références citées] ont été proposés qui se sont avérés présenter une activité anti-HIV.

La présente invention fournit de nouveaux composés analogues nucléosidiques d'anomérie β et de configuration L. Parmi ces L énantiomères, un petit nombre d'exemples de β-L-2', 3'-didéoxynucléosides ont été rapportés dans la littérature, mais selon des procédés de synthèse impliquant toujours une séparation des anomères α. [Brevet EP 352 248 A1 24 Jan. 1990 (CA: 113 (5), 41231 w (1990)]; Brevet EP 285 884 A2 12 Oct. 1988 (CA: 111 (3), 23911x (1989)] et/ou de leurs enantiomères D. [Brevet JP 02 069 469 A2, 8 mars 1990 (CA: 115 (1), 8560w (1991)]; Brevet JP 0 222 9192 A2, 11 septembre 1990 [CA: 114 ( II), 102709c (1991)]; Brevet JP 0206 9476 A2 8 mars 1990 [CA: 113 (11), 97977m (1990)]; L Kaulina, E Liepins, M. Lidaks et R.A. Zhuk, Khim. Geterstsikl - Soedin. (1), 101 (1982) [CA: 96 (17), 143 248e (1982)], obtenus de façon concommittante, de sorte que leur stéréospécificité ou leur pureté isomérique peut être mise en doute.

En particulier, le brevet EP 352 248 décrit un procédé de synthèse par condensation d'un sucre et d'une base purique ou pyrimidique.

Toutefois, dans le brevet EP 352 248, le composé sucre de départ possède un hydrogène ou un halogène en position 2', de sorte que la condensation avec la base B conduit à un mélange d'anomères α et β.

La présente invention fournit un procédé qui permet de préparer de façon stéréospécifique les composés β-L-2', 3'-didéoxy nucléosides. Il est apparu après évaluation de leur potentialité en tant qu'agent antiviral, plus particulièrement vis-à-vis du VIH, que certains de ces composés stéréoisomères étaient particulièrement actifs.

La présente invention a donc tout d'abord pour objet un procédé de préparation de composés 2' ou 3' déoxy et 2', 3'-didéoxy-β-L-pentofuranonucléosides de formule I: 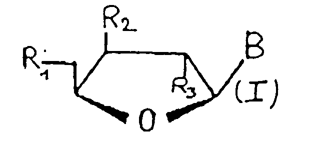 dans laquelle
- B représente une base purique ou pyrimidique;
- R₁ représente OH;
- R₂ et R₃ représentent indépendamment l'un de l'autre H ou OH;
- l'un au moins de R₂ et R₃ représente H;
caractérisé en ce que on réalise les étapes suivantes:
1) on condense un composé de formule (II) avc la base B pour obtenir le composé de formule (III) 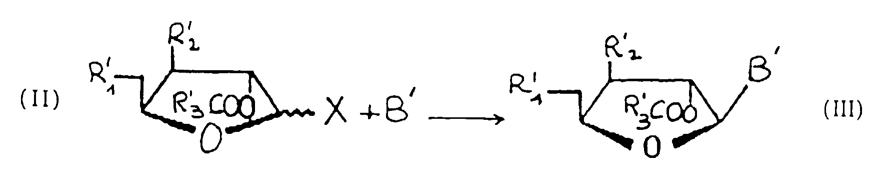 formules (II) et (III) dans lesquelles
   - R'₁ et R'₂ ont les significations données pour R₁ et R₂ excepté que quand R₁ et R₂ représentent OH, ledit groupe OH est protégé par un groupe protecteur tel qu'un groupe acyle, benzoyle, benzyle ou silyle,
   - R'₃ représente un groupe alkyl en C₁ à C₅ ou un radical phényl, éventuellement substitués,
   - X est un groupe partant tel que Cl, Br, I ou un groupe acyloxy ou alkoxy en C₁ à C₅,
   - B' est une base purique ou pyrimidique B éventuellement convenablement protégée,
2) l'on élimine le groupe R'₃ CO en position 2' par déacétylation de manière à obtenir un groupe OH et un composé de formule 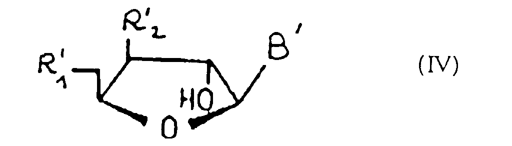
3) éventuellement, on élimine le groupe OH en position 2';
4) l'on déprotège, le cas échéant, les groupes R'₁ et R'₂ et la base B' de manière à obtenir les composés de formule (I).

La présence d'une protection acyle en position 2' entraîne un couplage stéréospécifique avec la base hétérocyclique conduisant stéréospécifiquement à l'anomère β de nucléoside au cours de la glycosylation selon les règle "trans" de Baker, car il induit la formation d'un acyloxonium intermédiaire.

N'importe quelle base hétérocyclique peut être condensée avec le sucre (II). On cite en particulier B représente l'une des bases l'adénine, la guanine, l'hypoxanthine, l'uracile, la thymine ou la cytosine, ces bases pouvant être substituées notamment par halogène en position 5 pour l'uracile et la cytosine.

Pour chaque type de base, les conditions de condensation glycosidique en synthèse nucléosidique sont multiples et bien connues de l'homme de l'art.

Au lieu de l'étape 3) d'élimination ci-dessus, on peut effectuer une substitution du groupe OH par un groupe N₃, F ou NH₂ avec alors une inversion de configuration sur le carbone considéré. On obtient alors des composés de formule (I) avec R₂ = N₃, F ou NH₂ avec la Configuration inverse de celle représentée à la formule (I).

De préférence, dans les composés (II) et (III), R'₃ représente un groupe alkyl en C₁ à C₅, de préférence CH₃.

Ainsi, on peut préparer les composés (II), dans lesquels on prépare le composé (II) di-O-acétylé en position 1, 2, dans lequel X et R'₃COO représentent un groupe O-acétyle par acétolyse du composé 1, 2 isopropylidène-L-xylofuranose de formule (V)

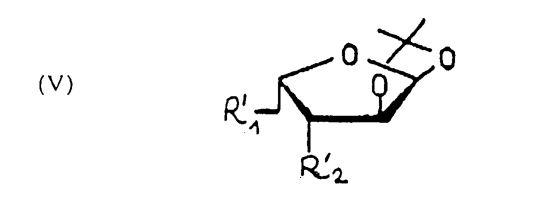

Cette réaction se fait en deux temps:
a) en milieu acide CH₃COOH 85% et H₂SO_{4,} puis
b) avec (CH₃ CO)₂ O dans la pyridine.

De préférence, R'₂ et R'₃ COO sont différents, en particulier R'₂ est un groupe O-benzoyl et R'₃ COO est un groupe 0-acyle.

Ainsi, il est possible de déprotéger sélectivement l'alcool en position 2' au moyen d'hydrazine hydratée à l'étape 2) ci-dessus.

Le Schéma I reprend les différentes étapes de synthèse de composés de formule (I) dans laquelle R₂ et R₃ représentent H ou OH en détaillant les conditions réactionnelles de l'homme de l'art. On peut introduire les composés de formule (I) sur les groupes N₃, F et NH₂ par substitution à la place du groupe OH avec inversion de configuration.

Dans le Schéma I, à partir du L-xylose commercial, deux voies de synthèse sont décrites, toutes deux impliquant l'obtention préalable d'un L-pentofuranose (composés 3 et 14) convenablement protégé et possédant en position 2 un groupement O-acyl participant induisant au cours des réactions de glycosylation la géométrie 1', 2'-Trans désirée pour le nucléoside obtenu (B.R. Baker, in The Ciba Foundation Symposium on the Chemistry and Biology of the Purines, G.E.W. Wrolstentiolme et C.M. O'Connor Eds, Churchill London, p. 120 (1957)). On parvient au sucre 3 et 14 à partir du L-xylose qui est transformé en 1, 2-isopropylidène-L-xylorurannose dont l'acétolyse conduit au dérivé di-O-acétylé en 1, 2.

Le composé 1 est obtenu en deux temps à partir d'acétone en présence de sulfate de cuivre puis en milieu acide.

Le composé 1 est mis à réagir avec du C₆ H₅ COCl dans de la pyridine pour obtenir le un intermédiaire dont les groupes OH en position 5' et 3' sont protégés par un benzoyle. Cet intermédiaire protégé est acétolysé en milieu acide (CH₃ COOH 85%, H₂ SO₄) puis en présence d'anydride (CH ₃ CO)₂ O dans la pyridine pour donner le composé 3.

La première voie de synthèse consiste à condenser le 1, 2-di-O-acétyl-3, 5-di-O-benzoyl-L-xylofurannose (3) avec une base hétérocyclique. La nature du groupement protecteur acétyle du sucre 3 en position 2' entraîne un couplage stéréospécifique conduisant à l'anomère β. le groupement protecteur en position 3' étant différent, on peut ensuite désacétyler sélectivement en position 2' au moyen d'hydrazine hydratée dans de la pyridine en milieu acide le β-L-xylofurannosyl nucléoside 4 totalement protégé obtenu, conduisant ainsi au composé 5. Ce dernier est ensuite transformé en son dérivé thiocarbonyle qui est soumis à une réaction de déoxygénation radicalaire de type Barton selon un procédé expérimental déjà utilisé en série D (M.J. Robins, D. Nadej, F. Hansske, J.S. Wilson, G. Gosselin, M.C. Bergogne, J.L. Imbach, J. Balzarini et F. De Clercq, Can. J. Chem. 66, 1258 (1988)), pour donner le composé 6. La réduction radicalaire de Barton consiste à substituer l'hydrogène de la fonction alcool par un groupement C(S)X(X = imidazole, phénoxy...) puis à réduire la fonction ROC(S)X par rupture homolytique au moyen de Bu₃SnH et d'AIBN. Le composé 6 est débenzoylé pour conduire au 2-déoxy-β-L-thréo-pentofuranosyl nucléoside 7. Une protection sélective de l'hydroxyle primaire 5' conduit au dérivé 8 qui est soumis à une désoxygénétion en 3' selon le procédé de type Barton. Finalement, le composé 9 résultant est déprotégé en 5' pour donner le 2', 3'-β-L-pentofuranosyl nucléoside 10.

La deuxième voie de synthèse implique la préparation préalable du 1, 2-di-O-acétyl-3-déoxy-5-O-benzoyl-L-erythro-pentofuranose (14), à ce jour inédit. Pour ce faire, le 1, 2-di-O-isopropylidène-α-L-xylofuranose (1), obtenu en deux étapes à partir du L-xylose et qui est un intermédiaire dans la synthèse du sucre 3 utilisé dans la première voie, est sélectivement benzoylé en position 5 pour donner le composé 11. Ce dernier est converti en son dérivé thiocarbonylé 12 qui est déoxygéné au moyen de triméthylsilylsilane (D.H.R. Barton, D.O. Jang et J. Cs. Jaszberenyi, Tetrahedron, 49, 2793 (1993)) pour conduire au composé 13. Ce composé 13 est déacétoné dans de l'acide acétique aqueux en présence d'acide sulfurique, et l'intermédiaire résultant n'est pas isolé, mais directement acétylé par de l'anhydride acétique dans de la pyridine pour conduire au sucre recherché 14. La condensation de 14 avec un aglycone purique ou pyrimidique conduit au nucléoside protégé 15 qui peut soit être totalement déacylé au moyen de méthylate de sodium ou d'ammoniac en solution dans le méthanol pour conduire au 3-déoxy-β-L-érytro-pentofuranosyl nucléoside 16, soit être sélectivement déacétylé en 2' au moyen de méthylate de sodium dans le THF pour donner le dérivé 17. Une réaction de déoxygénation selon Barton sur le dérivé 2'-thiocarbonylé de 17 conduit alors au dérivé 9, identique à celui obtenu dans la première voie de synthèse.

Pour illustrer la présente invention, la préparation (selon la première voie de synthèse) et la caractérisation de la 2'-3'-didéoxy-β-L-uridine (β-L-DDU), 10a) et celle de la 2', 3'-didéoxy-5-fluoro-β-L-uridine (β-L-5-fluoro-DDU), 10b) sont décrites dans les exemples qui vont suivre.

Pour préparer un composé de formule (I) dans lequel B est la cytosine, on peut selon la présente invention préparer un composé de formule (I) où B est l'uracile, puis transformer le dérivé d'uridine en dérivé de cytidine en transformant l'uracile en cytosine.

Les conditions expérimentales sont indiquées au Schéma II qui représente la transformation de la β-L-DDU et la β-L-5-fluoro-DDU. Ces deux composés ont été respectivement transformés (Schéma II) en 2', 3'-didéoxy-β-L-cytidine (β-L-DDC, 21a) et en 2', 3'-didéoxy-β-L-5-fluorocytidine (β-L-5-fluoro-DDC, 21b).

La β-L-DDU (10a) et la β-L-5-FDDU (10b) sont sélectivement acétylées en position 5' pour donner les composés 18a,b. Ces derniers sont convertis en leurs dérivés thioamides correspondants 19a, b par traitement avec le réactif de Lawesson aux reflux dans le dichloroéthane selon un procédé précédemment développé en série D de l'uridine (J.E. Starrett, Jr., D.R. Tortolani, D.C. Baker, M.T. Omar, A.K. Hebbler, J.A. Wos, J.C. Martin et M.M. Mansuri, Nucleosides Nucleotides, 9, 885 (1990)). Les composés 19a, b sont traités avec de l'ammoniac dans le méthanol soit à température ambiante, soit à 100°C pour conduire, respectivement, à la 2', 3'-didéoxy-β-L-4-thiouridine 20a et à son dérivé 5-fluoré 20b, ainsi qu'à la β-L-DDC (21a) et à la β-L-5-FDDC (21b) recherchées.

Alors que les isomères L sont considérés comme moins toxiques que les isomères D, car ils ne provoquent pas, semble-t-il, les mêmes mutations sur la reverse transcriptase, les résultats négatifs observés dans l'art antérieur, en ce qui concerne l'activité antivirale des composés du type 2', 3'-L-didéoxynucléosides (absence d'activité ou faible activité) pourraient être liés à un défaut de stéréospécificité.

En effet, la β-L-DCC (21a) et la β-L-5-F-DDC (21b) obtenues selon la présente invention ont été évaluées contre le VIH en cultures cellulaires (voir exemples ci-après) sur lesquelles ces deux molécules se sont révélées actives, avec notamment une très forte activité antivirale pour la β-L-5-FDDC.

En outre, la 2', 3'-didéoxy-β-L-5-fluoro-cytidine apparaît active sur des souches résistantes à l'AZT et à la névirapine, cette dernière faisant actuellement l'objet d'essais cliniques.

Les composés, dans lesquels l'un de R₂ ou R₃ est OH et l'autre est H, peuvent constituer des composés intermédiaires utiles dans la synthèse des dérivés 2', 3' didéoxy-β-L- nucléosides diversement substitués sur le sucre, notamment par des groupes N₃, F ou NH₂.

En particulier, les composés 2'-déoxy de configuration "threo" (voir en particulier Schéma I, les composés 7) et les composés 3'-déoxy de configuration "erythro" (voir en particulier Schéma I, les composés 16 par désoxygénation de leur hydroxyle conduisent aux β-L-2', 3'-didéoxynucléosides.

La présente invention a également pour objet des composés stéréoisomères β-L-pentofuranonucléosides stéréochimiquement pures répondant à la formule suivante 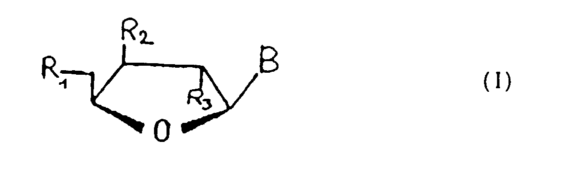 dans laquelle
- R₁ et B ont les significations données ci-dessus, et soit R₂ représente OH et R₃ représente H, soit R₂ représente H et R₃ représente OH.

En effet, les composés β-L-pentofuranonucléosides décrits dans la littérature comportent toujours une configuration inverse 3' ribo au lieu de 3' xylo comme selon la présente invention. Et il n'existe aucun exemple décrit dans la littérature de composés de formule (I) avec R₂ = H et R₃ = OH.

On cite en particulier les composés pour lesquels B représente l'uracile, la 5-fluoro-uracile, l'hypoxanthine, la 5-fluorocytosine la guanine ou l'adénine.

La présente invention a également pour objet des composés 2', 3' didéoxy β-L pentofuranonucléosides de formule (I) ci-dessus dans laquelle
. R₁ représente OH
. R₂ et R₃ représentent H et
. B représente l'uracile, la guanine, l'hypoxanthine, la 5 fluorouracile, la 5 fluorocytosine.

La présente invention a plus particulièrement pour objet un composé choisi parmi la β-L-ddU, β-L-5 fluoro-ddU, β-L-5-fluoro ddC.

Enfin, la présente invention a pour objet l'utilisation des composés selon l'invention à titre de médicament. Il peut s'agir, selon les cas, d'agents antibiotiques, antitumoraux ou d'agents anti-viraux, notamment anti-VIH. En particulier, en ce qui concerne les composés 2',3' didéoxynucléosides selon l'invention, ceux-ci sont plus particulièrement utiles comme agent anti-viral.

La présente invention a notamment pour objet l'application thérapeutique en chimiothérapie antirétrovirale de la β-L-5-F-DDC, plus particulièrement comme agent anti-VIH.

D'autres avantages et caractéristiques de la présente invention apparaîtront à la lumière des exemples qui vont suivre.

La Figure 1 représente le Schéma I.

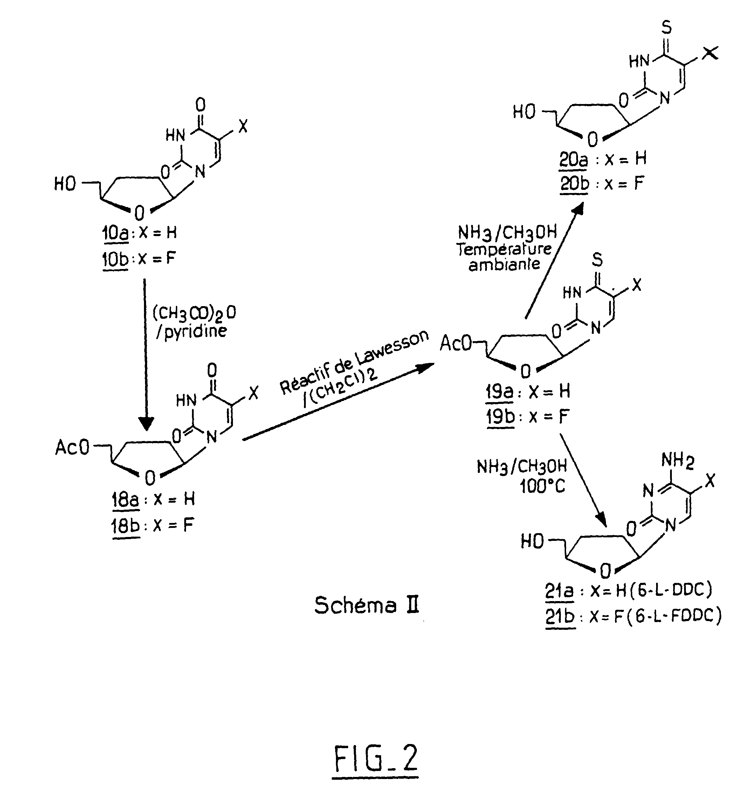

La Figure 2 représente le Schéma II.

### EXEMPLE 1 : Préparation du 1, 2-di-O-acétyl-3, 5-di-O-benzoyl-L-xylo-furanose (3)

Les modes opératoires et le matériel utilisés ont été décrits dans J. Chem. Soc., Perkin Trans. I 1943 (1992).

Le L-xylose a été acheté à Interchim, France.

Ce composé 3 est préparé en quatre étapes à partir du L-xylose sans purification des intermédiaires.

Selon le même protocole expérimental que décrit dans Gosselin et coll., Nucleic Acid Chemistry, Improved and New Synthetic Procedures, Methods and Techniques, PT4, L.B. Towsend and R.S. Tipan, eds, John Witey and Sons, Inc., 1991, p. 41.

Le mélange monomérique de 3 a été obtenu sous forme d'un sirop jaune pâle et une recristallisation avec de l'éthanol a conduit à l'anomère α pure (Rdt 26%) point de fusion 104-107°C.
Données RMN (DMSO, d): δ 2,06 et 2,10 (2 s, H.3 chacun, 2 COCH₃), 4,50 (m, H2, H5, 5'), 4,85 (m, 1H, H.1), 5.54 (dd, 1H, H-2, J = 4.6 et 5.79Hz (t, 1H, H.3, J = 6,3 Hz)), (6,43 d, 1H, H.1, J = 4,6 Hz), 7,5 - 8.0 (m, 10H, 2 CO C6H5) ; [α]²⁰ _{D} - 125,2°(C) 1,3 CHCl₃]; spectre de masse : (FAB > 0 matrice d'alcool 3-nitrobenzylique) m/z 443 [M + HJ⁺, 383 [383 [M - CH₃CO₂], 105 [C₆ H₅ C = 0]
Cacl. pour C₂₂ H₂₂ O₉ (442.41) : C : 62.44; H: 5.01 -
Trouvé : C : 62.28 et H : 5.04.

### EXEMPLE 2 : Préparation du 1 (2-0-Acétyl-3,5-di-O-benzoyl-β-L-xylo-furanosyl) Uracile (4)

A un mélange d'uracile (1.27 g ; 11,33 mmoles) et de sucre protégé (3) (5,0 g ; 11,30 mmoles), dans de l'acétonitrile anhydre (170 ml), on a ajouté successivement de l'hexaméthyl-disilazane (1,9 ml ; 9,01 mmoles), du triméthylchlorosilane (1,15 ml ; 9,06 mmoles) et du chlorure d'étain (IV) (1,59 ml ; 13,5 g mmoles). La solution limpide obtenue a été ajitée à température ambiante pendant 24 heures. Le mélange réactionnel a été concentré en un volume réduit, puis dilué avec du chloroforme (150 ml) puis lavé deux fois avec le même volume d'une solution d'hydrogénate de carbonate de sodium aqueux et finalement de l'eau. Les couches organiques ont été séchées sur du sulfate de sodium, filtré sur celite puis évaporé. Le produit obtenu a été purifié sur colonne de chromatographie de gel de silice [éluent : gradient de méthanol (0,4%) dans du chlorure de méthylène] pour donner le composé 4 pur (3,709, 66%).
CONDITIONS GENERALES ET INSTRUMENTATION MISES EN OEUVRES : Elles sont Identiques à celles rapportées par C. Périgaud, G. Gosselin et J.-L. Imbach, *J. Chem. Soc.*, *Perkin Trans*, 1, 1943 (1992).

### Exemple 3 : 2',3'-Dideoxy-β-L-uridine (β-L-DDU, 10a).

1-(2-Deoxy-β-L-*threo*-pentofuranosyl)uracile **(7a).**
Une solution de 1-(3,5-di-O-benzoyl-β-L-xylofuranosyl)uraclle **(6a)** (1,4 g : 3,21 mmol) dans du methanol ammoniacal (préalablement saturé à 10°C et hermétiquement fermé) (90 ml) est agitée durant deux jours à température ambiante. La solution est évaporée plusieurs fois avec du methanol sous pression réduite. Le matériel brut obtenu est dissous dans l'eau et la solution résultante est lavée plusieurs fois avec du chloroforme. La phase aqueuse est évaporée et le résidu est directement cristallisé dans le methanol pour donner 0,6 g (rendement 82 %) de **7a** pur : F : 165-167°C : RMN-¹H (DMSO-*d*₆), δ ppm = 11,23 (s, 1H, 3-NH), 7,92 (d, 1H H-6 ; J = 8,1 Hz), 6.04 (dd, 1H, H-1' ; J = 2,0 et 8,3 Hz), 5.26 (d, H, H-5 ; J = 8,1 Hz), 5.26 (d, 1H, OH-3' ; J = 3,2 Hz), 4,69 (t, 1H, OH-5': J = 5,3 Hz), 4,20 (m, 1H, H-3'), 3,81 (m, 1H, H-4'), 3,80-3,60 (m, 2H, H-5'et 5"), 2,6-2.5 (m, 1H. H-2', partiellement obscurci par DMSO-*d*₅), 1,85 (dd, 1H. H-2" ; J = 2,0 et 14,7 Hz) ; spectres de masse (matrice : glycerol-thioglycerol, 50:50, v/v):FAB>0 321 (M+glycérol+H]⁺, 229 [M+H]⁺, 117 [s]⁺ et 113 [BH₂]⁺ ; FAB<0 227 [M-H]⁻.

*Anal.* Calculé pour C₉H₁₂N₂O₅ (M = 228.21) : C 47.36 ; H 5.30 ; N 12,28. Trouvé : C 47,45 : H 5,46 ; N 12,12.

1-(5-O-Tertiobutyldiphenylsilyl-2-deoxy-β-L-*threo*-pentofuranosyl)uracile **(8a).**

A une solution de **7a** (0,6 g ; 2,63 mmol) dans de la pyridine anhydre (8 ml) on ajoute du chlorure de tertiobutyldiphenylsilane (0.9 ml ; 3,50 mmol). La solution est agitée durant 4 h à température ambiante, puis le solvant est évaporé sous pression réduite. De l'eau et du dichloromethane sont ajoutés. La phase organique est séparée, lavée successivement avec une solution aqueuse saturée d'hydrogenocarbonate de sodium et de l'eau, séchée sur sulfate de sodium et filtrée. Après évaporation à sec, le résidu est chromatographié sur une colonne de gel de silice [eluant : gradient par étape de méthanol (0-2 %) dans le dichloromethane] pour conduire à 1,2 g (98 %) de **8a** sous forme de mousse : RMN-¹H (DMSO-*d*₆) δ ppm : 11,26 (s, 1H, 3-NH), 7,78 (d, 1H, H-6 ; J = 8,2 Hz), 7,65-7,35 (m, 10 H, 2 C₆H₅], 6,09 (dd, 1H, H-1'; J = 1.9 et 6.4 Hz). 5,55 (d, 1H, H-5 ; J = 8.2 Hz), 5,30 (d,1H, OH-3'; J = 3.1Hz), 4,25 (m, H, H-3'), 4,05-3,95 (m, 2H, H-4' et 5'), 3,85-3,80 (m,1H, H-5"), 2.6-2.5 (m, 1H, H-2' partiellement obscurci par DMSO-*d*₅), 1,85 (dd, 1H, H-2" ; J = 1,9 et 16,5 Hz), 0,93 [s, 9H, (CH₃)₃C] ; spectre de masse (matrice : glycerol-thioglycerol, 50:50, v/v) FAB<0 465 [M-H]⁻ et 111 [B]⁻.

5'-O-Tertiobutyldiphenylsilyl-2',3'-dideoxy-β-L-uridine (**9a**).

A une solution de **8a** (1,1 g ; 2,36 mmol) dans de l'acetonitrile anhydre (66 ml) on ajoute du O-phenylchlorothionoformate (0,68 ml ; 5,02 mmol) et de la 4-dimethylaminopyridine (DMAP) (2,64 g ; 21,6 mmol). La solution est agitée durant une nuit à température ambiante puis le solvant est évaporé sous pression réduite. Du dichlorométhane et de l'eau sont ajoutés. La phase organique est séparée, puis successivement lavée avec une solution aqueuse refroidie 0,5 M d'acide chlorhydrique, de l'eau, une solution aqueuse saturée d'hydrogenocarbonate de sodium et à nouveau avec de l'eau avant d'être séchée sur sulfate de sodium, filtrée et évaporée à sec. Le résidu (1,9 g) est dissous dans du dioxane anhydre, la solution résultante est évaporée sous pression réduite, et cette opération est répétée trois fois pour conduire au dérivé thiocarbonate brut. Ce dernier est dissous dans du dioxane (28 ml) et traité avec de l'hydrure de tributyletain (1,57 ml ; 5,83 mmol) et de l'α,α'-azobisisobutyronitrile (AIBN) (0,12 g ; 0,73 mmol) à 90°C durant 2 h sous argon. Une quantité supplémentaire de Bu₃SnH (0.63 ml ; 2,3 mmol) et d'AIBN (50 mg ; 0,30 mmol) est rajoutée, et le chauffage est poursuivi durant 30 min. Après évaporation du solvant, du dichlorométhane et de l'eau sont rajoutés. La phase organique est séparée, séchée sur sulfate de sodium et évaporée à sec. Le résidu est chromatogrphié sur une colonne de gel de silice [éluant : gradient par étape de méthanol (0-5 %) dans le dichloromethane] pour conduire à 0,54 g (rendement 51 %) de **9a** pur qui cristallise dans l'ether éthylique : F = 145-147°C ; UV (EtOH 95] λmax 264 nm, λ min 235 nm ; RMN-¹H (DMSO-*d*₆) δ ppm : 11.29 (s, 1H, 3-NH). 7,74 (d, 1H, H-6), 7,65-7,40 (m, 10 H, 2 C₆H₅), 5,99 (dd, 1H, H-1' ; J = 3,1 et 7,4 Hz), 5,21 (d, 1H, H-5 ; J = 8,1 Hz), 4,15-4,00 (m, 1H, H-4'), 3,94 (dd, 1H, H-5' ; J = 3,0 et 11,3 Hz), 3,75 (dd, 1H, H-5" ; J = 4,0 et 11,3 Hz), 2,45-2,20 (m, 1H, H-4'), 2,10-1,85 (m, 3H; H-2", 3' et 3"), 0,99 [s,9H, (CH₃)₃C] ; spectre de masse (matrice ; glycerol-thioglycerol, 50:50, v/v) FAB>0 451 [M+H]⁺, 339 [s]⁺ et 113 [BH₂]⁺.

2',3'-Dideoxy-β-L-uridine (β-L-ddU : **10a**).
Le composé **9a** (0,25 g ; 0,55 mmol) est dissous dans du tetrahydrofurane (1,1 ml) et une solution 1,1 M de fluorure de tetra-*n*-butylammonium dans le THF (0,55 ml) est ajoutée. La solution est agitée durant 2 h à température ambiante, puis évaporée sous pression réduite. Du dichlorométhane et de l'eau sont rajoutés, la phase aqueuse est évaporée à sec. Le résidu est chromatographié sur une colonne de gel de silice [eluant : gradient par étape de méthanol (0-5 %) dans le dichlorométhane] pour donner 41 mg (rendement 35 %) de **10a** pur qui cristallise dans le dichlorométhane : F = 120-121°C ; UV (EtOH 95) max 262 nm, λ min 231 nm ; RMN-¹H (DMSO)*d*₆) δ ppm : 11,24 (s, 1H, 3-NH), 7,93 (d, 1H, H-6 ; J = 8.1 Hz), 5,93 (dd, 1H, H-1' ; J = 3,4 et 6,7 Hz), 5,57 (d, 1H, H-5 ; J = 8,1 Hz), 5.02 (triplet mal résolu, 1H, OH-5'), 4,05-3,95 (m, 1H, H-4'), 3,70-3,40 (m, 2H, H-5'et 5" ; après échange D₂O : 3,63 ppm (dd, 1H, H-5' ; J = 3,4 et 12,1 Hz) et 3,49 (dd, 1H, H-5" ; J = 4,0 et 12,1 Hz), 2,35-2.20 (m, 1H, H-2'), 2,0-1,65 (m, 3H, H-2", 3' et 3") ; spectres de masse (matrice : glycerol-thioglycerol, 50:50, v/v) : FAB>0 425 [2M+H]⁺, 213 [M + H]⁺, 113 [BH₂]⁺ et 101 [s]⁺ ; FAB<0 211 [M-H]⁻ et 111 [B]⁻.

### Exemple 4 : 2',3'-Dideoxy-β-L-5-fluorouridine (β-L-5-FDDU, 10b)

1 - (2-Deoxy-β-L-*threo*-pentofuranosyl)-5-fluorouracile (**7b**).

Une solution de 1-(3,5-di-O-benzoyl-2-deoxy-β-L-xylofuranosyl)-5-fluorouracile (**6b**) (0.25 g, 0,55 mmol) dans du méthanol ammoniacal (25 ml) est agitée durant une nuit à température ambiante. La solution est évaporée sous pression réduite et le résidu est évaporé plusieurs fois avec du méthanol. Le matériel brut obtenu est dissous dans de l'eau et la solution résultante est lavée plusieurs fois avec du chloroforme. La phase aqueuse est évaporée et le résidu est directement cristallisé dans le méthanol pour donner 115 mg (rendement 85 %) de **7b** pur : F = 198-200°C ; UV (EtOH 95) λ max 267 nm (ε, 8500), λ min 233 nm (ε, 2100); RMN-¹H (DMSO-*d*₆) δ ppm = 11,79 (s, 1H, 3-NH), 8,16 (d, 1H, H-6 ; J = 7,4 Hz), 6,05 (dd, 1H, H-1'; J = 1,8 et 6,5 Hz), 5,38 (d, 1H, OH-3' ; J = 3.3 Hz), 4,73 (t, 1H, OH-5'; J = 5,4 Hz), 4,30-4,20 (m, 1H, H-3'), 3,85-3,60 (m, 3H, H-4',5' et 5"), 2,60-2,50 (m, 1H, H-2' partiellement obscurci par DMSO-*d*₅), 1,90 (dd, 1H H-2" ; J = 1,8 et 14,7 Hz) ; spectres de masse (matrice : glycérol-thioglycérol, 50:50, v/v) : FAB>0 339 [M+glycerol+H]⁺, 247 [M+H]⁺, 131 [BH₂]⁺ et 115 [s]⁺ ; FAB<0 245 [M-H]⁻ et 129 [B]⁻.
*Anal.* Calculé pour C₉H₁₁N₂O₅F (M = 246,20): C 43,90 ; H 4,51 ; N 11,38 ; F 7,72. Trouvé : C 43,60 ; H 4,57 ; N 11,22 ; F 7,40.

1-(5-O-Monomethoxytrityl-2-deoxy-β-L-*threo*-pentofuranosyl)-5-fluorouracile (**8b**).

A une solution de **7b** (1,30 g ; 5,28 mmol) dans de la pyridine anhydre (60 ml), on ajoute du 4-methoxytriphenylchloromethane (1,96 g ; 6,35 mmol). La solution est agitée durant 48 h à température ambiante, puis le solvant est évaporé sous pression réduite. De l'eau et du dichlorométhane sont ajoutés. La phase organique est séparée, lavée successivement avec une solution aqueuse saturée d'hydrogénocarbonate de sodium et de l'eau, séchée sur sulfate de sodium et filtrée. Après évaporation à sec, le résidu est chromatographié sur une colonne de gel de silice [eluant : gradient par étape de méthanol (0-5 %) dans le dichlorométhane] pour conduire à 2,6 g (rendement 95 %) de **8b** sous forme de mousse : RMN-¹H (DMSO-*d*₆) δ ppm : 11,82 (s, 1H, 3-NH), 7,92 (d, 1H,H-6 ; J = 7,3 Hz), 7,5-6,8 (m,14H, mMTr), 6,11 (d, 1H, H-1' ; J = 7,9 Hz), 5,35 (d, 1H, OH-3' ; J = 3,1 Hz), 4,20-4,15 (m, 1H, H-3'), 4,15-4,10 (m, 1H, H-4'), 3,72 (s, 3H, OCH₃), 3,40-3,10 (m, 2H, H-5' et 5"), 2,60-2,45 (m,1H, H-2' partiellement obscurci par DMSO-*d*₅), 1,90 (d, 1H, H-2" ; J = 14,7 Hz) ; spectre de masse (matrice : glycérol-thioglycérol, 50:50, (v/v) : FAB<0 1553 [3M-H]⁻, 1035 [2M-H]⁻, 517 [M-H]⁻, 245 [M-monomethoxytrityl]⁻ et 129 [B]⁻.

5'-O-Monomethoxytrityl-2',3'-dideoxy-β-L-5-fluorouridine (**9b**).

Ce composé est préparé selon un procédé analogue à celui employé lors de la synthèse de **9a**. Ainsi, **8b** (2.8 g : 5,40 mmol) est mis en réaction avec du O-phenyl chlorothionoformate (1.5 ml ; 11,08 mmol) et de la DMAP (5,97 g, 48,85 mmol) dans de l'acétonitrile anhydre (250 ml) pour donner, après traitement, un résidu qui est traité par du Bu₃SnH (3,75 ml ; 13,93 mmol) et de l'AIBN (0,28 g ; 1,70 mmol) dans du dioxane (95 ml) durant 2 h sous argon. Après traitement, le résidu est chromatographié sur une colonne de gel de silice [ eluant : gradient par étape de méthanol (0-2 %) dans le dichlorométhane] pour conduire à 1.6 g (rendement 59 % de **9b** sous forme de mousse : RMN-¹H (DMSO-*d*₆) δ ppm : 11,84 (s, 1H, 3-NH), 7,90 (d, 1H, H-6 ; J = 6,8 Hz), 7,50-6,80 (m, 14 H, mMTr), 5,95 (d, 1H, H-1' ; J = 5,9 Hz), 4,20-4.10 (m, 1H, H-4'), 3,72 (s, 3H, OCH₃), 3,40-3,15 (m, 2H, H-5' et 5" partiellement obscurci par H₂O), 2,40-2,20 (m, 1H, H-2'), 2,20-1,85 (m, 3H, H-2", 3' et 3") ; spectre de masse (matrice : glycérol-thioglycérol, 50 : 50, v/v): FAB<0 1003 [2M-H]⁻, 501 [M-H]⁻, 229 [M-monomethoxytril]⁻, 129 [B].

2',3'-Dideoxy-β-L-5-fluorouridine (β-L-5-FDDU : **10b**).

Le composé **9b** (1,6 g ; 3,18 mmol) est dissous dans de l'acide acétique aqueux à 80 % et la solution est agitée à température ambiante durant 2 h. Après évaporation des solvants, le résidu est coévaporé plusieurs fois avec un mélange toluène-méthanol. Une chromatographie sur colonne de gel de silice [eluant : gradient par étape de méthanol (0-5 %) dans le dichlorométhane] conduit à 0,6 g (rendement 82 %) de **10b** sous forme de mousse : RMN-¹H (DMSO-*d*₆) δ ppm : 11,76 (s,1H, 3-NH), 8,38 (d, 1H, H-6 ; J = 7,5 Hz), 5,89 (dd, 1H, H-1' ; J = 2,0 et 4,0 Hz), 5,20 (t, 1H, OH-5' ; J = 5,0 Hz); 4,10-4,00 (m, 1H, H-4'), 3,80-3,65 [m, 1H, H-5'; après échange D₂O: 3,69 ppm, dd, J = 2,8 et 12,3 Hz], 3,60-3,50 [m, 1H, H-5" ; après échange D₂O : 3,50 ppm, dd, J = 3,3 et 12,3 Hz), 2,35-2,20 (m, 1H, H-2'), 2,10-1,80 (m, 3H, H-2", 3' et 3") ; spectres de masse (matrice : glycérol-thioglycérol, 50 : 50, v/v) : FAB>0 231 [M+H]⁺, 131 [BH2]⁺, 101 [s]⁺ ; FAB<0 229 [M-H]⁻, [B]⁻.

### Exemple 5 : 2',3'-Dideoxy-b-L-cytidine (β-L-DDC, 21a).

A une solution de β-L-DDU (**10a**) (0,4 g ; 1,88 mmol) dans de la pyridine anhydre (6 ml) on ajoute à 0°C de l'anhydride acétique (0,27 ml ; 2.9 mmol) et le mélange réactionnel est agité 1 h à 0°C, puis 5 h à température ambiante. On rajoute ensuite de l'eau glacée et l'on extrait deux fois avec du chloroforme. La phase organique est séchée sur sulfate de sodium, filtrée, coévaporée plusieurs fois avec du toluène et évaporée à sec pour conduite à 0,55 g d'un résidu correspondant à la 5'-O-acétyl β-L-DDU (**18a**) suffisamment pure (ccm) pour être directement utilisée dans l'étape suivante. A une solution du résidu dans du dichlorethane anhydre (49 ml) on ajoute le réactif de Lawesson (Aldrich, Art. 22,743-9 ; 0,64 g ; 1,6 mmol) et le mélange est porté à reflux sous argon durant 2 h. Après évaporation des solvants, le résidu est chromatographié sur colonne de gel de silice [eluant : gradient par étape de méthanol (0-1 %) dans le dichlorométhane) pour donner 0,55 g de 5'-O-acetyl-β-L-4-thiouridine (**19a**) suffisamment pure (ccm) pour être directement utilisée dans la dernière étape. Le résidu est dissous dans du méthanol ammoniacal (11 ml), et la solution est chauffée à 100°C pendant 3 h dans un autoclave. Le mélange est refroidi, évaporé à sec et le résidu est chromatographié sur colonne de gel de silice [eluant : gradient par étape de méthanol (0-12 %) dans le dichlorométhane] pour donner 0,33 g (rendement 83 %) de β-L-DDC (**21a**) pure qui cristallise dans l'éthanol : F = 220-222 °C ; UV (EtOH 95) max 273 nm, λmin 252 nm ; RMN-¹H (DMSO-*d*₆) δ ppm = 7,89 (d, 1H, H-6 ; J = 7,4 Hz), 7,15-6,95 (d large, 2H, NH₂), 5,91 (dd, 1H, H-1' ; J = 3,0 et 6,5 Hz), 5,66 (d, 1H, H-5 ; J = 7,4 Hz), 4,99 (t, 1H, OH-5' ; J = 5,2 Hz), 4,05-3,95 (m, 1H, H-4'), 3,60-3,70 (m, 1H, H-5' ; après échange D₂O : dd, 3,64 ppm, J = 3,6 et 12,0 Hz], 3,60-3,50 (m, 1H, H-5" : après échange D₂O : dd, 3,50 ppm, J = 4,1 et 12,0 Hz]. 2,30-2,15 (m, 1H, H-2'), 1,9-1,65 (m, 3H, H-2", 3'et 3") ; [α]_{D}²⁰ -103,6 (c 0,8, méthanol) ; spectres de masse (matrice : glycérol-thioglycérol, 50 : 50, v/v) : FAB>0 423 [2M+H]⁺, 304 [M+glycérol+H]⁺, 212 [M+H]⁺, 112 [BH₂]⁺, 101 [s]⁺ ; FAB<0 210 [M-H]⁻.

*Anal.* Calculé pour C₉H₁₃N₃O₃ (M = 211.21) ; C 51,18 ; H 6,20 ; N 19,89. Trouvé : C 51,34 ; H 6,25 N 20,12.

### Exemple 6 : 2',3'-Dideoxy-β-L-5-fluorocytidine (β-L-5-FDDC, 21b)

A une solution de β-L-5-FDDU (**10b**) (0,55 g ; 2.39 mmol) dans de la pyridine anhydre (10 ml) on ajoute à 0°C de l'anhydride acétique (0.34 ml ; 3,60 mmol) et le mélange réactionnel est agité 1 h à 0 °C puis 3 h à température ambiante. Une quantité supplémentaire d'anhydride acétique (0,22 ml ; 2,33 mmol) est rajoutée et l'agitation est poursuivie pendant 3 h à température ambiante. On rajoute ensuite de l'eau glacée et l'on extrait deux fois avec du chloroforme. La phase organique est séchée sur sulfate de sodium, filtrée, coévaporée plusieurs fois avec du toluène, et évaporée à sec pour conduire à 0,67 g d'un résidu correspondant à la 5'-O-acetyl-β-L-5-FDDU (**18b**) suffisamment pure (ccm) pour être directement utilisée dans l'étape suivante. A une solution du résidu dans du dichloroéthane anhydre (67 ml) on ajoute le réactif de Lawesson (0,60 g ; 1,48 mmol) et le mélange est porté au reflux sous argon. Deux quantités supplémentaires de réactif de Lawesson sont rajoutées, respectivement après 2 h (0.41 g ; 1,01 mmol) et 3 h (0,20 g ; 0,49 mmol) de reflux. Après évaporation des solvants, le résidu est chromatographié sur colonne de gel de silice [eluant : gradient par étape de méthanol (0-2 %) dans le dichlorométhane] pour donner 0,48 g de 5'-O-acetyl-β-L-5-fluoro-4-thiouridine (**19b**) suffisamment pure (ccm) pour être directement utilisée dans la dernière étape. Le résidu est dissous dans du méthanol ammoniacal (12 ml) et la solution est chaufée à 100°C pendant 3 h 30 dans un autoclave. Le mélange est refroidi, évaporé à sec et le résidu est chromatographié sur colonne de gel de silice [eluant : gradient par étape de méthanol (0-8 %) dans le dichlorométhane] pour donner 0,27 g (rendement 51 %) de β-L-5-FDDC (**21b**) pure qui cristallise dans l'acétate d'éthyle ; F = 158-160°C ; UV (EtOH 95) λ max 281 nm (ε, 8400) et 237 nm (ε, 8500); min 260 nm (ε, 5700) et 225 nm (ε, 7800) ; RMN-¹H (DMSO-*d*₆) δ ppm 8,28 (d, 1H, H-6 ; J = 7,4 Hz), 7,7-7,4 (d large, 2H, NH₂), 5,83 (dd mal résolu, 1H, H-1'), 5,16 ( t, 1H, OH-5' ; J = 5,1 Hz), 4,05-3,95 (m, 1H, H-4'), 3,8-3,70 (m,1H, H-5' ; après échange D₂O : dd, 3,71 ppm, J = 2,7 et 12,3 Hz], 3,60-3,50 [m, 1H, H-5" ; après échange D₂O : dd, 3,52 ppm ; J = 3,3 et 12,3 Hz], 2,35-2,15 (m, 1H, H-2'), 1,95-1,75 (m, 3H, H-2", 3' et 3") ; [α]_{D}²⁰ -80,0 (-c 1,0, DMSO) ; spectres de masse (matrice : alcool 3-nitrobenzylique) FAB>0 230 [M+H]⁺ ; 130 [BH₂]⁺ et 101 [s]⁺ ; FAB<0 228 [M-H]⁻.
*Anal.* Calculé pour C₉H₁₂N₃FO₃ (M = 229,21) : C 47,16 ; H 5,28 ; N 18,33, F 8,29. Trouvé : C 46,90 ; H 5,28 ; N 18,07 ; F 8,17.

Les composés de l'invention ont été soumis à des essais pharmacologiques montrant leur intérêt dans le traitement de maladies virales.

### Evaluation de l'activité anti VIH 1 sur diverses lignées cellulaires.

### VIH = virus de l'immunodéficience humaine.

La réplication du VIH-1 (isolat LAI) dans les lignées cellulaires est mesurée par un dosage de la réverse transcriptase (RTase) dans le surnageant de culture après 5 jours d'infection. Cette activité traduit la présence de virus libéré par les cellules. Après l'adsorption du virus, les composés testés sont ajoutés à différentes concentrations dans le milieu de culture.
L'activité antivirale est exprimée par la concentration la plus faible de composé qui diminue la production de RTase d'au moins 50 % (ED₅₀).

L'effet toxique sur les cellules non infectées est apprécié par une réaction colorimétrique basée sur la capacité des cellules vivantes à réduire le bromure de 3-(4,5-diméthylthiazol-2-yl)-2,5 diphenyltetrazolium en formazan après 5 jours d'incubation en présence de différentes concentrations des composés. Les résultats sont exprimés par la concentration la plus faible de composé qui provoque une inhibition d'au moins 50 % de la formation de formazan (CD₅₀).

La β-L-DDC (**21a**) et plus encore la β-L-5FDDC (**21b**) ont une ED₅₀ marquée sur le VIH-1 et le VIH-2 comme indiqué ci-après

| **Composés :** | | **21a** | **21b** | **AZT** | **DDC** |
|---|---|---|---|---|---|
| CEM-SS/VIH-1 LAI | ED₅₀ | 3 10⁻⁷ M | 3.8 10⁻⁸ M | 2.5 10⁻⁹ M | 3.5 10⁻⁸ M |
| | CD₅₀ | 8.3 10⁻⁵ M | 9 10⁻⁵ M | > 10⁻⁴ M | 6 10⁻⁵ M |
| | | | | | |
| PBM/VIH 1 III B | ED₅₀ | 3.5 10⁻⁷ M | 3 10⁻⁸ M | 1.1 10⁻⁹ M | 4 10⁻⁸ M |
| | CD₅₀ | 10⁻⁴ M | 10⁻⁴ M | 7 10⁻⁵ M | 7 10⁻⁵ M |
| | | | | | |
| PBMC/VIH-2 D 194 | ED₅₀ | 3.5 10⁻⁸ M | 4.5 10⁻⁸ M | 1 10⁻⁹ M | 2 10⁻⁹ M |
| | CD₅₀ | 10⁻⁴ M | 10⁻⁴ M | 8 10⁻⁵ M | 2.5 10⁻⁵ M |

De plus cette activité anti VIH-1 est confirmée sur diverses autres lignées cellulaires : MT-4 (ED₅₀ : **21a** 1.5 10⁻⁵ M,**21b** 2.4 10⁻⁶ M; Ref: AZT 2.7 10⁻⁸ M, DDC 2.5 10⁻⁶ M) U 937 (ED₅₀ : **21a** 1.5 10⁻⁷ M, **21b** 4.2 10⁻¹⁰ M; Ref: AZT 4 10⁻¹⁰ M, DDC 3.8 10⁻¹⁰ M) CEM TK⁻ (ED₅₀ : **21a** 9.5 10⁻⁸ M,**21b** 9.5 10⁻⁸ M; Ref: AZT > 10⁻⁴ M, DDC 2.5 10⁻⁶ M)

Enfin ces composés présentent aussi une activité anti VIH-1 sur les lignées résistantes à l'AZT et à la Nevirapine
CEM-SS/VIH-1 Nevirapine résistant (ED₅₀ : **21a** 10⁻⁶ M, **21b** 7.2 10⁻⁷ M ; Ref: AZT 7.5 10⁻⁶ M, DDC 1.2 10⁻⁷ M)
MT2/VIH-1 AZT résistant (Larder) (ED₅₀ : **21a** 3.5 10⁻⁷ M, **21b** 2 10⁻⁷ M ; Ref: AZT 7 10⁻⁶ M, DDC 2.2 10⁻⁷ M)

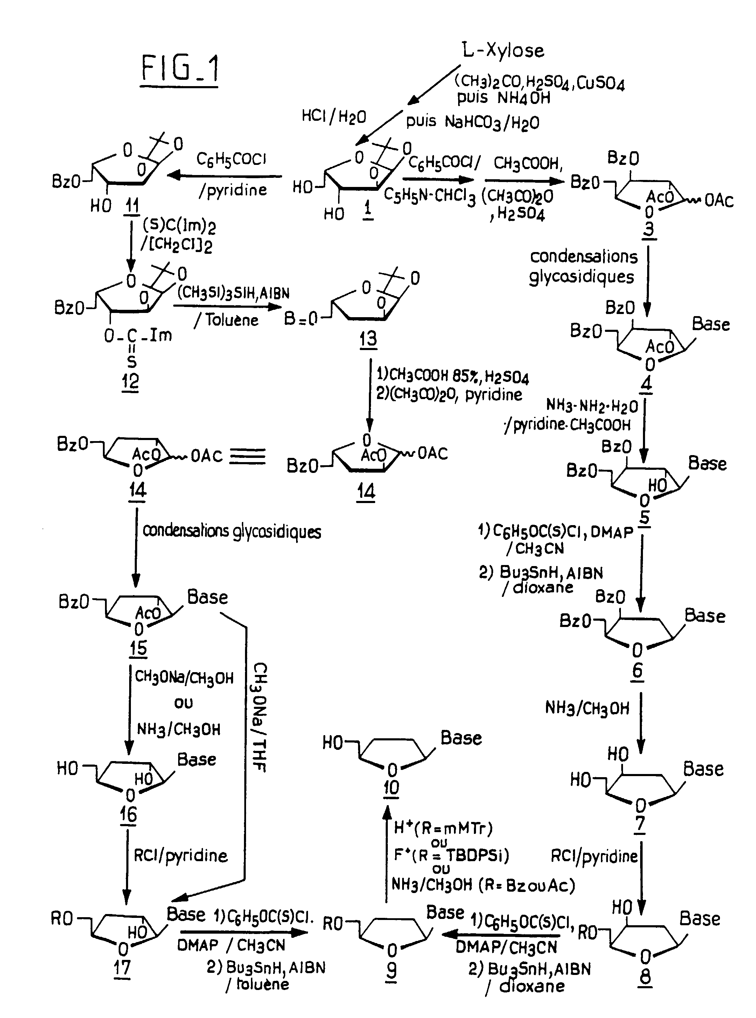

### Légende Figure 1

Schéma I : Bases = purines ou pyrimidines, éventuellement convenablement protégées ; R = Benzoyl (Bz), acétyl (Ac), monométhoxytrityl (mMTr), ou tertiobutyldiphenylsilyl.

## Revendications

1. Procédé de préparation de composés 2' ou 3' déoxy et 2', 3'-didéoxy-β-L-pentofuranonucléosides de formule I: 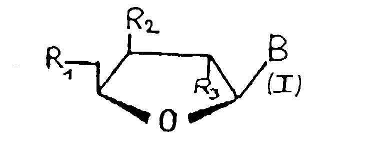 dans laquelle
- B représente une base purique ou pyrimidique;
- R₁ représente OH;
- R₂ et R₃ représentent indépendamment l'un de l'autre H ou OH;
- l'un au moins de R₂ et R₃ représente H,
caractérisé en ce que on réalise les étapes suivantes:
1) on condense un composé de formule (II) avec la base B pour obtenir le composé de formule (III) suivant le schéma: 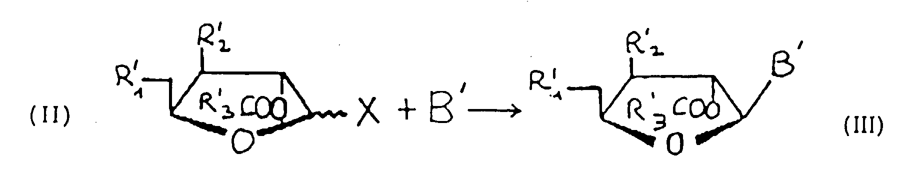 formules (Il) et (III) dans lesquelles
- R'₁ et R'₂ ont les significations données pour R₁ et R₂ excepté que quand R₁ et R₂ représentent OH, ledit groupe OH est protégé par un groupe protecteur tel qu'un groupe acyle, benzoyle, benzyle ou silyle,
- R'₃ représente un groupe alkyl en C₁ à C₅ ou un radical phényl, éventuellement substitués,
- X est un groupe partant tel que Cl, Br, I ou un groupe acyloxy ou alkoxy en C₁ à C₅,
- B' est une base purique ou pyrimidique B éventuellement convenablement protégée,
2) on élimine le groupe R'₃ CO en position 2' par déacétylation de manière à obtenir un groupe OH et un composé de formule 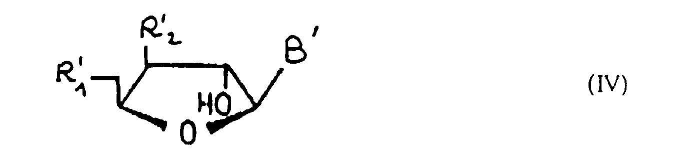
3) éventuellement, on élimine le groupe OH en position 2', et
4) on déprotège, le cas échéant, les groupes R'₁ et R'₂ et la base B' de manière à obtenir les composés de formule (I).

2. Procédé selon la revendication 1, caractérisé en ce que, dans les composés (II) et (III), R'₃ représente un groupe alkyl en C₁ à C₅, de préférence CH₃.

3. Procédé selon la revendication 1 ou 2, caractérisé en ce que on prépare le composé (II) di-O-acétylé en position 1, 2, dans lequel X et R'₃ COO représentent un groupe O-acétyle, par acétolyse du composé 1, 2 isopropylidène-L-xylofurannose de formule (V)

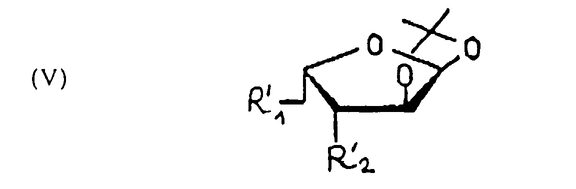

4. Procédé selon l'une des revendications 1 à 3, caractérisé en ce que R'₂ et R'₃ COO sont différents, en particulier R'₂ est un groupe O-benzoyl et R'₃ est un groupe alkyle.

5. Procédé selon l'une des revendications 1 à 4, caractérisé en ce qu'on prépare les composés de formule (I) où R₂ et R₃ représentent H ou OH.

6. Procédé selon l'une des revendications 1 à 4, caractérisé en ce que B représente l'une des bases adénine, guanine, hypoxanthine, uracile, thymine ou cytosine, ces bases pouvant être substituées notamment par un halogène en position 5 pour la cytosine et l'uracile.

7. Procédé de préparation d'un composé de formule (I) où B est la cytosine selon l'une des revendications 1 à 6, caractérisé en que on prépare un composé de formule (I) dans lequel B est l'uracile selon le procédé des revendications 1 à 6 et l'on transforme le dérivé d'uridine en dérivé de cytidine en transformant l'uracile en cytosine.

8. Composés stéréoisomères β-L-pentofuranonucléosides répondant à la formule suivante 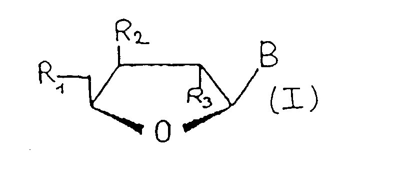 dans laquelle
- B a la signification donnée dans l'une des revendications 1 et 6, R₁ représente OH et,
- soit R₂ représente OH et R₃ représente H,
- soit R₂ représente H et R₃ représente OH.

9. Composés selon la revendication 8 caractérisé en ce que B représente l'uracile, la 5-fluoro-uracile, l'hypoxanthine, la 5-flurocytosine, la guanine ou l'adénine.

10. Composés stéréoisomères 2', 3' - didéoxy-β-L pentofuranonucléosides répondant à la formule (I) 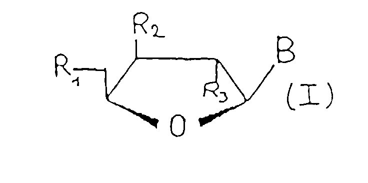 dans laquelle :
. R₁ représente OH
. R₂ et R₃ représentent H et
. B représente l'uracile, la guanine, l'hypoxanthine, la b fluorocytosine, la fluorocytosine.

11. Composé selon la revendication 10 choisi parmi la β-L-ddU, β-L-5 fluoro-ddU, β-L-5-fluoro ddC.

12. Utilisation des composés selon l'une des revendications 8 à 11 pour obtenir un médicament.

13. Utilisation des composés selon l'une des revendications 8 à 11 pour obtenir un médicament anti-viral.

14. Utilisation des composés selon l'une des revendications 8 à 11 pour obtenir un médicament anti-viral utile pour le traitement du SIDA.

15. Utilisation de la β-L-5-fluoro ddC selon la revendication 14 pour obtenir un agent antiviral.

16. Utilisation de la β-L-5 fluoro ddC selon la revendication 15 pour obtenir un agent anti-VIH.

## Patentansprüche

1. Verfahren zur Herstellung von 2'- oder 3'-Desoxy- und 2',3'-Didesoxy-β-L-pentofuranonucleosiden der Formel I: 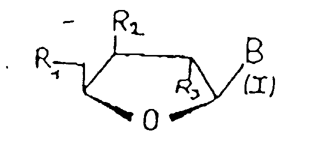 worin
- B eine Purin- oder Pyrimidinbase darstellt;
- R₁ die Bedeutung von OH hat;
- R₂ und R₃ unabhängig voneinander H oder OH bedeuten;
- mindestens eines von R₂ und R₃ die Bedeutung von H hat,
dadurch gekennzeichnet, daß man folgende Stufen durchführt:
1) man kondensiert eine Verbindung der Formel (II) mit der Base B zur Bildung einer Verbindung der Formel (III) nach folgendem Schema: 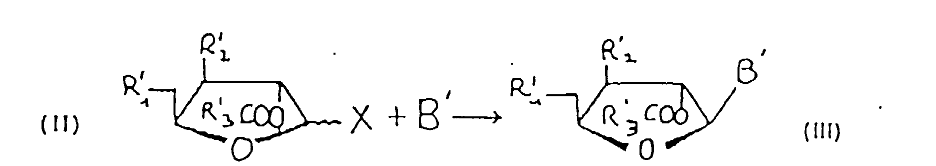 wobei in den Formeln (II) und (III)
- R'₁ und R'₂ die für R₁ und R₂ angegebenen Bedeutungen haben, mit der Ausnahme, daß wenn R₁ und R₂ die Bedeutung von OH haben, die OH-Gruppe durch eine Schutzgruppe, wie eine Acyl-, Benzoyl-, Benzyl- oder Silylgruppe geschützt ist,
- R'₃ eine Alkylgruppe mit C₁ bis C₅ oder einen Phenylrest, die gegebenenfalls substituiert sein können, darstellt,
- X eine abspaltbare Gruppe, wie Cl, Br, I oder eine Acyloxy- oder Alkoxygruppe mit C₁ bis C₅ bedeutet,
- B' eine Purin- oder Pyrimidinbase darstellt, wobei B gegebenenfalls zweckmäßig geschützt ist,
2) man entfernt die R'₃ CO-Gruppe in 2'-Stellung durch Desacetylieren derart, daß eine OH-Gruppe und eine Verbindung der Formel 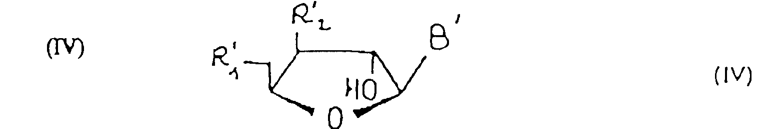 gebildet werden,
3) man entfernt gegebenenfalls die OH-Gruppe in 2'-Stellung und
4) man führt gegebenenfalls eine Schutzgruppenabspaltung der Gruppen R'₁ und R'₂ sowie der Base B' derart durch, daß die Verbindungen der Formel (I) erhalten werden.

2. Verfahren nach Anspruch 1, dadurch gekennzeichnet, daß in den Verbindungen (II) und (III) R'₃ eine Alkylgruppe mit C₁ bis C₅, vorzugsweise CH₃, darstellt.

3. Verfahren nach Anspruch 1 oder 2, dadurch gekennzeichnet, daß man die in 1, 2-Stellung di-O-acetylierte Verbindung (II), worin X und R'₃ COO eine O-Acetylgruppe darstellen, durch Acetolyse der Verbindung 1, 2-Isopropyliden-L-xylofurannose der Formel (V) 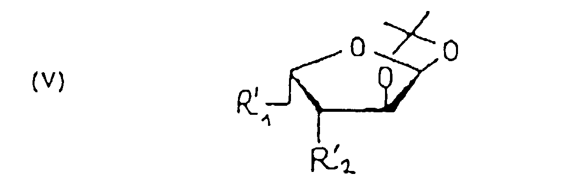 herstellt.

4. Verfahren nach einem der Ansprüche 1 bis 3, dadurch gekennzeichnet, daß R'₂ und R'₃ COO unterschiedlich sind, und insbesondere R'₂ eine O-Benzoylgruppe und R'₃ eine Alkylgruppe sind.

5. Verfahren nach einem der Ansprüche 1 bis 4, dadurch gekennzeichnet, daß man die Verbindungen der Formel (I) herstellt, worin R₂ und R₃ die Bedeutung von H oder OH haben.

6. Verfahren nach einem der Anspruche 1 bis 4, dadurch gekennzeichnet, daß B eine der Basen Adenin, Guanin, Hypoxanthin, Uracil, Thymin oder Cytosin ist, wobei diese Basen substituiert sein können, insbesondere durch ein Halogen in der 5-Stellung für das Cytosin und das Uracil.

7. Verfahren zur Herstellung einer Verbindung der Formel (I), worin B das Cytosin ist, gemäß einem der Ansprüche 1 bis 6, dadurch gekennzeichnet, daß man eine Verbindung der Formel (I), worin B das Uracil ist, gemäß dem Verfahren der Ansprüche 1 bis 6 herstellt und das Uridinderivat in ein Cytidinderivat überführt, wobei man das Uracil in Cytosin umwandelt.

8. Stereoisomere β-L-Pentofuranonucleosid-Verbindungen der folgenden Formel 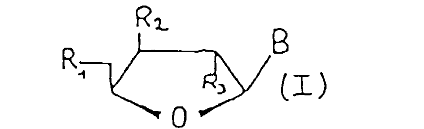 worin
- B die in einem der Ansprüche 1 bis 6 angegebene Bedeutung hat und R₁ die Bedeutung von OH hat und
- entweder R₂ die Bedeutung von OH und R₃ die Bedeutung von H hat,
- oder R₂ die Bedeutung von H und R₃ die Bedeutung von OH hat.

9. Verbindungen nach Anspruch 8, dadurch gekennzeichnet, daß B Uracil, 5-Fluorouracil, Hypoxanthin, 5-Fluorocytosin, Guanin oder Adenin darstellt.

10. Stereoisomere 2', 3'-Didesoxy-β-L-pentofuranonucleoside der Formel (I) 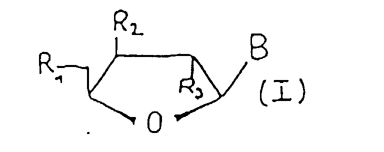 worin
- R₁ die Bedeutung von OH hat,
- R₂ und R₃ die Bedeutung von H haben und
- B Uracil, Guanin, Hypoxanthin, b Fluorocytosin, Fluorocytosin darstellt.

11. Verbindung nach Anspruch 10, ausgewählt aus β-L-ddU, β-L-5-Fluoro-ddU, β-L-5-Fluoro ddC.

12. Verwendung der Verbindungen nach einem der Ansprüche 8 bis 11 zur Herstellung eines Arzneimittels.

13. Verwendung der Verbindungen nach einem der Ansprüche 8 bis 11 zur Herstellung eines antiviralen Arzneimittels.

14. Verwendung der Verbindung nach einem der Ansprüche 8 bis 11 zur Herstellung eines antiviralen Arzneimittels, das zur Behandlung von AIDS geeignet ist.

15. Verwendung von β-L-5-Fluoro ddC nach Anspruch 14 zur Herstellung eines antiviralen Mittels.

16. Verwendung von β-L-5-Fluoro ddC nach Anspruch 15 zur Herstellung eines Anti-HIV-Mittels.

## Claims

1. Method for the preparation of 2'- or 3'-deoxy- and 2',3'-dideoxy-β-L-pentofuranonucleoside compounds of formula I: 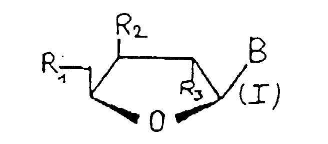 in which
- B represents a purine or pyrimidine base;
- R₁ represents OH;
- R₂ and R₃ represent, independently of each other, H or OH;
- at least one of R₂ and R₃ represents H;
characterized in that the following steps are carried out:
1) a compound of formula (II) is condensed with the base B in order to obtain the compound of formula (III) according to the scheme 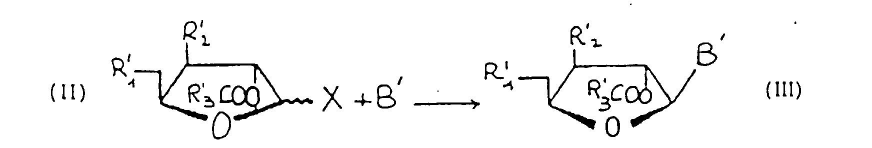 in which formulae (II) and (III)
- R'₁ and R'₂ have the meanings given for R₁ and R₂ except that when R₁ and R₂ represent OH, the said OH group is protected by a protecting group such as an acyl, benzoyl, benzyl or silyl group,
- R'₃ represents a C₁ to C₅ alkyl group or a phenyl radical, which are optionally substituted,
- X is a leaving group such as Cl, Br, I or a C₁ to C₅ acyloxy or alkoxy group,
- B' is a purine or pyrimidine base B which is optionally appropriately protected,
2) the R'₃ CO group at the 2' position is removed by deacetylation so as to obtain an OH group and a compound of formula 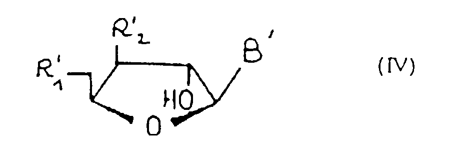
3) optionally, the OH group at the 2' position is removed; and
4) where appropriate, the R'₁ and R'₂ groups and the B' base are deprotected so as to obtain the compounds of formula (I).

2. Method according to Claim 1, characterized in that in the compounds (II) and (III), R'₃ represents a C₁ to C₅ alkyl group, preferably CH₃.

3. Method according to Claim 1 or 2, characterized in that the compound (II), di-O-acetylated at the 1,2 position, in which X and R'₃COO represent an O-acetyl group, is prepared by acetolysis of the 1,2-isopropylidene-L-xylofuranose compound of formula (V)

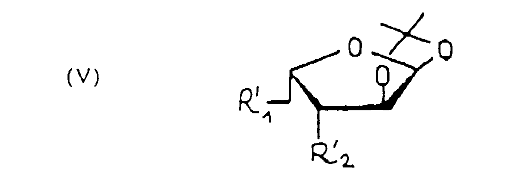

4. Method according to one of Claims 1 to 3, characterized in that R'₂ and R'₃COO are different, in particular R'₂ is an O-benzoyl group and R'₃ is an alkyl group.

5. Method according to one of Claims 1 to 4, characterized in that the compounds of formula (I) are prepared in which R₂ and R₃ represent H or OH.

6. Method according to one of Claims 1 to 4, characterized in that B represents one of the adenine, guanine, hypoxanthine, uracil, thymine or cytosine bases, it being possible for these bases to be substituted especially by a halogen at the 5 position for cytosine and uracil.

7. Method for the preparation of a compound of formula (I) in which B is cytosine according to one of Claims 1 to 6, characterized in that a compound of formula (I) is prepared in which B is uracil according to the method of Claims 1 to 6 and the uridine derivative is converted to a cytidine derivative by converting uracil to cytosine.

8. Stereoisomeric β-L-pentofuranonucleoside compounds corresponding to the following formula 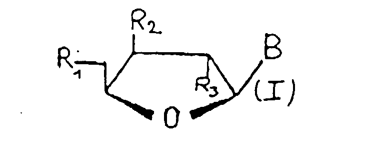 in which
- B has the meaning given in one of Claims 1 and 6, R₁ represents OH and,
- either R₂ represents OH and R₃ represents H,
- or R₂ represents H and R₃ represents OH.

9. Compounds according to Claim 8, characterized in that B represents uracil, 5-fluorouracil, hypoxanthine, 5-fluorocytosine, guanine or adenine.

10. Stereoisomeric 2',3'-dideoxy-β-L-pentofuranonucleoside compounds corresponding to the formula (I) 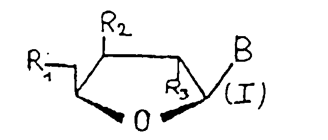 in which:
. R₁ represents OH
. R₂ and R₃ represent H and
. B represents uracil, guanine, hypoxanthine, b fluorocytosine, fluorocytosine.

11. Compound according to Claim 10, which is chosen from β-L-ddU, β-L-5 fluoro-ddU, β-L-5-fluoro ddC.

12. Use of the compounds according to one of Claims 8 to 11, to obtain a drug.

13. Use of the compounds according to one of Claims 8 to 11, to obtain an antiviral drug.

14. Use of the compounds according to one of Claims 8 to 11, to obtain an antiviral drug which is useful for the treatment of AIDS.

15. Use of β-L-5-fluoro ddC according to Claim 14, to obtain an antiviral agent.

16. Use of β-L-5-fluoro ddC according to Claim 15, to obtain an anti-HIV agent.
